(19) Europäisches Patentamt
European Patent Office
Office européen des brevets

(11) Publication number: **0 095 665**
**B1**

# (12) EUROPEAN PATENT SPECIFICATION

(45) Date of publication of patent specification: **06.08.86**

(21) Application number: **83104929.1**

(22) Date of filing: **19.05.83**

(51) Int. Cl.[4]: **G 01 N 33/532,** C 07 K 15/00, A 61 K 39/395, C 07 D 207/40, C 07 H 17/06

(54) **Labelled conjugates of ethosuximide, ethosuximide immunogens, antibodies thereto, immunoassay method employing the antibodies and reagent means, test kit and test device for the immunoassay method.**

(30) Priority: **01.06.82 US 383599**

(43) Date of publication of application:
**07.12.83 Bulletin 83/49**

(45) Publication of the grant of the patent:
**06.08.86 Bulletin 86/32**

(84) Designated Contracting States:
**CH DE FR GB IT LI NL SE**

(56) References cited:
**GB-A-1 511 571**

(73) Proprietor: **MILES LABORATORIES, INC.**
**1127 Myrtle Street**
**Elkhart Indiana 46514 (US)**

(72) Inventor: **Buckler, Robert T.**
**23348 May Street**
**Edwardsburg, MI 49112 (US)**
Inventor: **Wong, Raphael C.**
**21 Elder Glen**
**Irvine, CA 92714 (US)**

(74) Representative: **Jesse, Ralf-Rüdiger, Dr. et al**
**Bayer AG Konzernverwaltung RP**
**Patentabteilung**
**D-5090 Leverkusen 1 Bayerwerk (DE)**

**EP 0 095 665 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

1. Background of the invention

Field of the invention

This invention relates to novel ethosuximide derivatives pertaining to immunoassays for determining ethosuximide in liquid media such as biological fluids. Such derivatives include ethosuximide immunogens used to stimulate production of antibodies to ethosuximide in host animals by conventional techniques. Also provided are labeled ethosuximide conjugates used as reagents, along with the ethosuximide antibodies, in particularly preferred immunoassays. Intermediates in the synthesis of the aforementioned immunogens and labeled conjugates are also provided.

Ethosuximide [2-ethyl-2-methylsuccinimide, c.f., *The Merck Index,* 9th ed., p. 493(1976)] is an anticonvulsant drug of the formula;

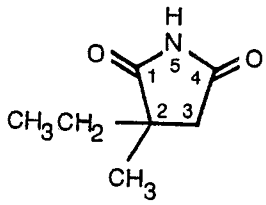

As with most anti-convulsants ethosuximide possesses a low therapeutic index and has clearly separable ranges of drug concentration in blood in which it is ineffective, therapeutic, or toxic, respectively. At sufficiently high concentration, the drug is potentially toxic and is eliminated from the body at a rate which can vary within a wide range from individual to individual so that the same dose can yield an ineffective, a therapeutic, or a toxic concentration depending on the subject. Thus, it is desirable to monitor the concentration of the drug in the blood in order to establish correct dosage in each patient.

2. Description of the prior art

The determination of ethosuximide by immunoassay is well known. Commercial immunoassay is well known. Commercial immunoassay test kits are available for measuring serum and plasma ethosuximide levels. Homogeneous immunoassays are for determining various analytes including drugs are described in U.S. Pat. Nos. 4,279,992; 4,238,565; and 3,817,837 and in British Pat. No. 1,552,607.

Immunogen conjugates, comprising ethosuximide coupled to conventional immunogenic carrier materials, useful in stimulating the production of antibodies to the drug in animals are suggested in the literature. British Patent Spec. No. 1,511,571 proposes ethosuximide immunogens wherein the drug is linked at its N-5 position or through one of the C-2 alkyl substituents to the carrier. Japanese Provisional Spec. No. 56-046,820 (Derwent No. 44742D/25) describes ethosuximide immunogens wherein the drug is linked through the C-2 ethyl substituent with a particular phenylene linking group. The state-of-the-art of preparing antibodies to haptens such as drugs is represented by Weinryb *et al, Drug Metabolism Reviews 10*:271(1979); Playfair *et al, Br. Med. Bull. 30*:24(1074)) Broughton *et al, Clin. Chem. 22*:726(1976); and Butler, *J. Immunol. Meth. 7*:1(1975).

Labeled conjugates, comprising the analyte or a derivative or other analog thereof, coupled to a labeling substance are variously described in the literature, e.g., the aforementioned U.S. Pat. No. 4,279,992 and U.S. Pat. Nos. 4,182,856; 4,259,233; and 4,292,425 wherein the label is the fluorogenic enzyme substrate ß-galactosyl-umbelliferone.

SUMMARY OF THE INVENTION

The present invention uniquely provides reagents for use in ethosuximide immunoassays involving the coupling to or derivatization of the drug at the C-3 psotion. The ethosuximide immunogen of the present invention, comprising the haptenic drug covalently linked at its 3-position to an immunogenic carrier material, stimulates the production of antibodies having advantageous cross-reactivity characteristics. By coupling the drug at the 3-position where no substituents appear in the parent drug, the immunogen conjugate is prepared without modifying any functional or distinguishing groups on the drug.

In a preferrred embodiment, the present invention provides novel intermediates in the preparation of the 3-substituted ethosuximide reagents. Also provided are an improved immunoassay method and reagent means for the determination of ethosuximide with the use of the novel antibodies of the present invention. The present invention also provides labeled ethosuximide conjugates for particularly preferred embodiments of such immunoassay.

DESCRIPTION OF THE PREFERRED EMBODIMENTS

The present invention, in all of its interrelated embodiments, is focused on preparation of 3-substituted ethosuximide derivatives which can then be used to form immunogens by coupling them to conventional carrier materials, and subsequently used to obtain ethosuximide antibodies, or can be used to form labeled conjugates which serve as the detection reagents in ethosuximide immunoassays.

*Ethosuximide Immunogens*

The present immunogens have the general formula:

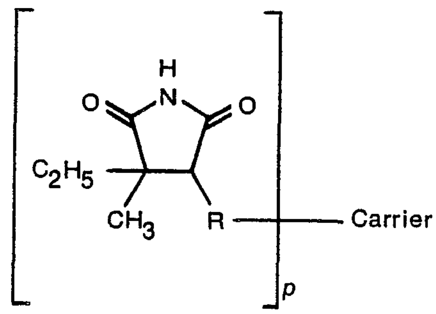

wherein R is an appropriate linking group, Carrier is a conventional immunogenic carrier material, and *p* is the number of ethosuximide moieties conjugated to the carrier. The number *p* is sometimes referred to as the epitopic density of the immunogen and in the usual situation will be on the average from 1 to about 50, more normally from 1 to about 25. Optimal epitopic densities, considering the ease and reproducibility of synthesis of the immunogen and antibody response, fall between about 2 and about 20, more usually between 5 and 15.

The immunogenic carrier material can be selected from any of those conventionally known. In most cases, the carrier will be a protein or polypeptide, although other such materials such as carbohydrates, polysaccharides, lipopolysaccharides, nucleic acids and the like of sufficient size and immunogenicity can likewise be used. For the most part, immunogenic proteins and polypeptides will have molecular weights between 5,000 and 10,000,000, preferably greater than 15,000, and more usually greater than 50,000. Generally, proteins taken from one animal species will be immunogenic when introduced into the blood stream of another species. Particularly useful proteins are albumins, globulins, enzymes, hemocyanins, glutelins, proteins having significant nonproteinaceous constituents, e.g., glycoproteins, and the like. The albumins and globulins of molecular weight between 30,000 and 200,000 are particularly preferrred. Further reference for the state-of-the-art concerning conventional immunogenic carrier materials and techniques for coupling heptens thereto may be had to the following: Parker, *Radioimmunoassay of Biologically Active Compounds,* Prentice-Hall (Englewood Cliffs, New Jersey USA, 1976); Butler, *J. Immunol. Meth. 7*:1-24 (1975); Weinryb and Shroff, *Drug Metab. Rev. 10*:271-283 (1975); Broughton and Strong, *Clin. Chem. 22*:726-732 (1976); and Playfair *et al, Br. Med. Bull. 30*:24-31 (1974).

Appropriate 3-substituted ethosuximide derivatives are couplable to immunogenic carrier materials according to well known techniques. For example, 3-carboxylated ethosuximide derivatives can be coupled to immunogenic carriers having amino groups by standard peptide bond-forming reactions such as described in *Peptides,* ed. Goodman and Meienhofer, John Wiley & Sons (New York 1977) p. 6 *et seq.* 3-Thiolated ethosuximide derivatives can be attached to carriers having thiol groups by the disulfide interchange reaction [Martin *et al, Biochem. 20*:4229 (1981)]. Alternatively, a thiol group can be introduced into one of the reactants and a maleimido group into the other and the two coupled together in a manner similar to that decribed by Liu *et al, Biochem. 18*:690 (1979). 3-Aminated ethosuximide derivatives can be coupled to carriers having carboxyl groups by amide bond-forming reactions such as those involving carbodiimides [Aherne *et al, Brit. J. Clin. Pharm. 3*:561 (1976)], mixed anhydrides [Erlanger *et al, Methods Immunology and Immunochemistry,* ed. Williams and Chase, Academic Press (New York 1967) p. 149], and the acid azide and active ester reactions [Kopple, *Peptides and Amino Acids,* W. A. Benjamin (New York 1966). See also *Clin. Chem. 22*:726 (1976). Similarly, the amino-derivatives can be coupled to carriers having amino groups using conventional bifunctional reagents such as *bis*-isocyanates, *bis*-imidoesters, and glutaraldehyde [*Immunochem. 6*:53 (1969)]. See also Kopple, *supra,* and Lowe and Dean *Affinity Chromatography,* John Wiley & Sons (New York 1974). A multitude of other coupling techniques are available to those of ordinary skill in the art for joining the various 3-ethosuximide derivatives of the present invention with conventional immunogenic carrier materials.

Particularly preferred are the ethosuximide immunogens of the formula

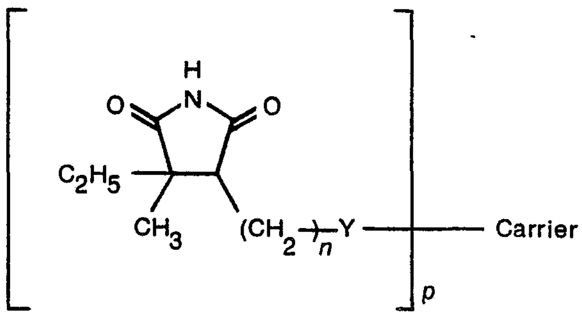

wherein Y is an amide group, i.e., —NHCO—, Carrier is an immunogenic protein or polypeptide, *n* is an integer from 1 through 10, and *p* is on the average from 1 to the number of available amide coupling sites on the carrier material, and preferably is as defined above. The amide coupling group may be oriented in either of the two possible ways, with the nitrogen atom in the amide group being from carrier amino groups and the carbon atom being from an approrpiate ethosuximide derivative (e.g., a carboxylic acid), with *p* then representing the average number of coupled amino groups in the carrier (and preferably is as defined above), or with the nitrogen atom being from an appropriate ethosuximide derivative (e.g., an amino derivative) and the carbon atom being from carrier carboxyl groups, with *p* then representing the average number of coupled carboxyl groups in the carrier (and preferably is again as defined above).

*Ethosuximide Antibodies*

Preparation of specific antibodies using the present immunogen conjugates may follow any conventional technique. Numerous aspects of inducing antibody formation, for example reference may be made to Parker *Radioimmunoassay of Biologically Active Compounds,* Prentice-Hall (Englewood Cliffs, New Jersey, USA, 1976). In the usual case, a host animal such as rabbit, goat, mouse, guinea pig, or horse is injected at one or more of a variety of sites with the immunogen conjugate, normally in mixture with an adjuvant. Further injections are made at the same site or different sites at regular or irregular intervals thereafter with bleedings being taken to assess antibody titer until it is determined that optimal titer has been reached. The host animal is bled to yield a suitable volume of specific antiserum. Where desirable, purification steps may be taken to remove undesired material such as nonspecific antibodies before the antiserum is considered suitable for use in performing actual assays.

The antibodies can also be obtained by somatic cell hybridization techniques, such antibodies being commonly referred to as monoclonal antibodies. Reviews of such monoclonal antibody techniques are found in *Lymphocyte Hydridomas,* ed. Melchers *et al,* Springer-Verlag (New York 1978), *Nature 266*:495(1977), *Science 208*:692 (1980), and *Methods in Enzymology 73 (Part B)*:3-46(1981).

*3-Substituted Ethosuximide Derivatives*

The drug ethosuximide *(1)* (2-ethyl-2-methylsuccinimide) is synthesized as outlined in Scheme 1. Methyl ethyl ketone *(2)* is condensed with ethyl cynaoacetate *(3)* to give the unsaturated cyano ester *(4)*. This reacts with cyanide ion to give the dinitrile *(5)*, which, when treated with sulfuric acid, undergoes de-carbethoxylation and ring closure to give ethosuximide *(1)* [Morel and Foucaud, *Bull. Soc. Chim. (France),* 3123(1970)].

SCHEME 1

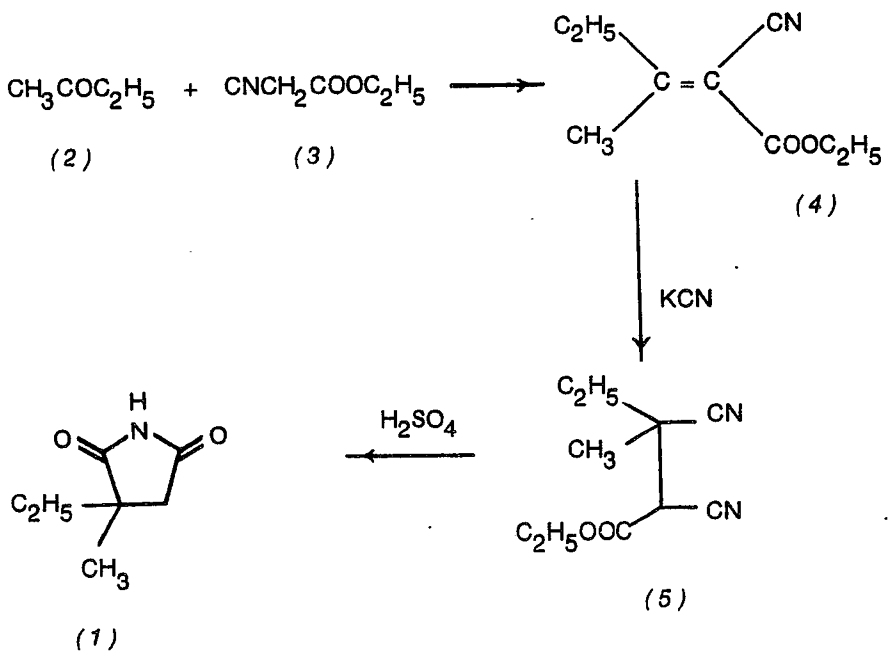


(2) (3) (4) (5) (1)

The synthesis of 3-substituted ethosuximide derivatives can be accomplished by a variety of routes. Usually, such derivatives will be prepared by an appropriate variation of the above synthesis of the parent drug or by direct alkylation or the like of the parent drug. In the former case, a wide variety of functional groups can be attached to the 3-position of ethosuximide.

For example, as outlined in Scheme 2, the intermediate *(5)*, generated by the addition of cyanide ion to *(4)*, can be reacted directly with a reagent X—R′—Z *(6)* where X is a leaving group (such as halogen,

SCHEME 2

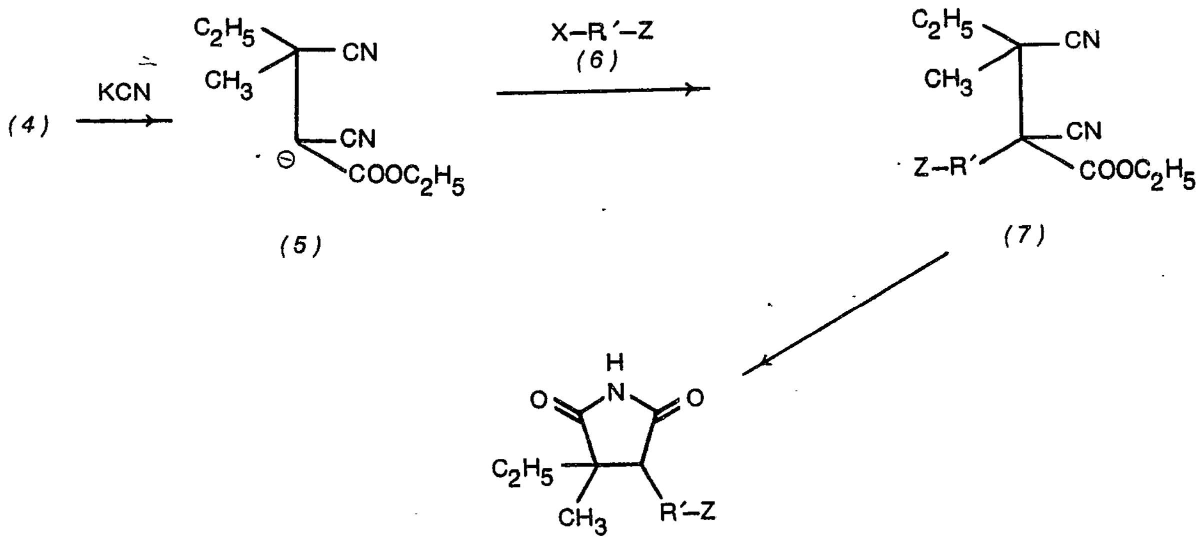


(4) (5) (7) (8)

*p*-toluenesulfonyl, methansuflonyl, etc.) and Z is a functional group, or a suitably protected form of functional group, capable of forming covalent bonds with proteins or other immunogenic carriers, or with labels used in immunoassays. Thus, Z may be COOH, COOR (where R = lower alkyl), CN, maleimido, thiol, [or a suitably protected form of thiol group; cf, *Protective Groups in Organic Chemistry,* Greene, John

Wiley & Sons, (New York, 1981), p 193], suitable protected amino group [cf, Greene, *supra,* p. 218; *The Peptides,* Vol. 3, Gross and Meienhofer, eds., Academic Press (New York, 1981), p. 3; and Hardy, *Chem. Anal.,* 617 (1979)]. The structure of linking group R′ is only restricted by the requirement that it be stable to the reaction conditions used to attach X—R′—Z to intermediate *(5).*

As mentioned previously, another route to the 3-substituted ethosuximide derivatives of the present invention involves direct alkylation of the parent drug. Ethosuximide *(1)* can be converted to the di-anion *(9)* by reaction with 2 equivalents of lithium diisopropylamide (LDA) in a mixture of tetrahydrofuran (THF) and hexamethylphosphoric triamide (HMPA).

SCHEME 3

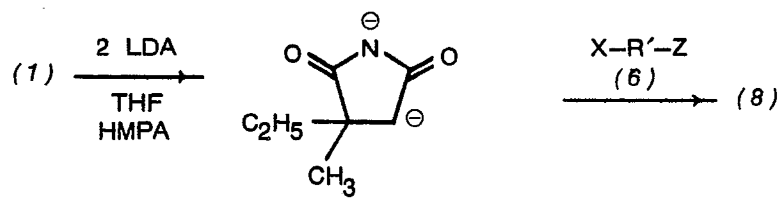


*(9)*

The di-anion *(9)* can be alkylated with X—R′—Z *(6)* discussed above to give 3-substituted derivatives *(8).*

When derivative *(8)* is coupled by a suitable technique to an immunogenic carrier material to form an ethosuximide immunogen, the residue of —R′—Z presents in the conjugate becomes referred to herein as linking group R. Thus, it can readily be seen that one skilled in the art has a wide variety of linking groups R′ and R that can be introduced to the derivatives and conjugates of the present invention. Exemplary of such choices are linear and branched alkylenes comprising from 1 to as many as 15, more usually 10 or less, and normally less than 6, carbon atoms (e.g., methylene, ethylene, *n*-propylene, *iso*-propylene, *n*-butylene, and so forth). In addition, such alkylenes can contain other substituent groups such as cyano, amino (including substituted amino), acylamino, halogen, thiol, hydroxyl, carbonyl groups, carboxyl (including substituted carboxyls such as esters, amides, and substituted amides). The linking groups R′ and R can also contain or consist of substituted or unsubstituted aryl, aralkyl, or heteroaryl groups (e.g., phenylene, phenethylene, and so forth). Additionally, such linkages can contain one or more heteroatoms selected from nitrogen, sulfur and oxygen in the form of ether, ester, amido, amino, thio ether, amidino, sulfone, or sulfoxide. Also, such linkages can include unsaturated groupings such as olefinic or acetylenic bonds, imino, or oximino groups. Preferably R and R′ will be a chain, usually an aliphatic group, comprising between 1 and about 20 atoms, more usually between 1 and 10, excluding hydrogen, of which between 0 and 5 are heteroatoms selected from nitrogen, oxygen, and sulfur. Particularly preferred are the derivatives wherein R′ is $-(CH_2)_n-$ with *n* being an integer from 1 through 10 and wherein Z is amino or carboxyl. Therefore, the choice of linking groups R′ and R is not critical to the present invention and may be selected by one of ordinary skill taking normal precautions to assure that stable compounds are produced.

Similarly, the terminal functional group Z can vary widely, although amino, carboxyl, thiol, hydroxyl, and maleimido will be preferred. Examples of synthetic routes available to obtain 3-ethosuximide derivatives having such preferred terminal functional groups follow:

(a) 3-(Carboxy-functionalized) ethosuximides

Following the procedure of Scheme 2 or 3, intermediates *(5)* or *(9)* can be alkylated with reagent *(6)* where X is a leaving group as previously defined and Z is a carboxyl group or a substituent which can be converted into a carboxyl group. The only restriction is that the leaving group X be attached to R′ in such a way that it will undergo a typical nucleophilic displacement [cf, Gould, *Mechanism and Structure in Organic Chemistry,* Holt, Rinehart and Winston, Inc., (New York 1959), p. 250 *et seq*].

For example, *(5)* can be alkylated with the potassium salt of 3-chloropropionic acid as described by Buckler *et al, J. Med. Chem. 21:* 1254 (1978) to give, after cyclization, *(8)* (R′ = $CH_2CH_2$, Z = COOH). Intermediate *(9)* can be alkylated with 2-(3-chloropropyl) benzyl chloride to give *(7)* wherein

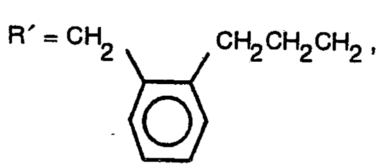

and Z = Cl

which upon reaction with cyanide ion and treatment with acid will produce the carboxyl ethosuximide *(8)* wherein

R' = 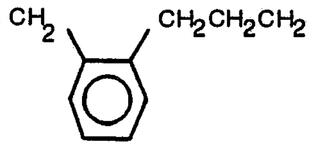 and Z = COOH

cf. Buckler *et al, Europ, J. Med. Chem.* 12:463(1977). Alternatively, either intermediate *(5)* or *(9)* can be alkylated with ethyl *omega*-bromoalkanoates Br—$(CH_2)_n$—$COOC_2H_5$ (except where $n = 2$) to give intermediates which upon treatment with acid give 3-substituted ethosuximide derivatives when R' = $(CH_2)_n$ and Z = COOH. Ethyl *omega*-bromoalkanoates $n = 1$ through 4 are commercially available (Aldrich Chemical Co., Milwaukee, WI); $n = 5$ through 9 are known compounds; cf Barger *et al, J. Chem. Soc.,* (1973) 714; Salmon-Legagneur, *Bull. Soc. Chem. (France)* (1956) 411; and Linnell and Vora, *J. Pharm, Pharmacol. 4*: 55 (1952).

(b) 3-(Amino-functionalized) ethosuximides)

With X and R' defined as in section (a) preceding, intermediates *(5)* or *(9)* can be reacted with reagent *(6)* where Z is an amino group, or more preferred, a blocked amino group or a functional group which can be transformed into an amino group.

For example, *(5)* can be alkylated with N-(*omega*-bromoalkyl)phthalimides to give intermediates *(7)* wherein

R' = $(CH_2)_n$ and Z = 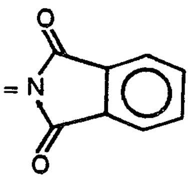

which upon treatment with acid followed by hydrazine will give the amino-ethosuximide *(8)* (R' = $(CH_2)_n$, Z = $NH_2$). N(*omega*-bromoalky) phthalimides $n = 2$—4 are commercially available. Analogs $n = 5$—9 are known compounds; cf, Dirscherl and Weingarten, *Ann. 574*: 131 (1951); Muller and Krauss, *Montash. 61*: 219 (1932); Elderfield *et al, J. Amer. Chem. Soc. 68*: 1568 (1946); Donahue *et al, J. Org. Chem. 22*:68 (1957).

Di-anion *(9)* can be alkylated with allyl chloride to give *(8)* (R' = $CH_2$, Z = CH=$CH_2$). Oxidation of this with $OsO_4/NaIO_4$ will give the aldehyde *(8)* (R' = $CH_2$, Z = CHO) [Pappo *et al, J. Org. Chem. 21*:478 (1956)] which can be reductively alkylated to give the primary amine *(8)*, Z = $NH_2$ [Borch *et al, J. Amer. Chem. Soc. 93*:2897 (1971)].

(c) 3-(Thiol-functionalized ethosuximides

With X and R' as described above, a thiol group can be attached to the 3-position of ethosuximide by reacting a 3-(amino-substituted) compound with N-succinimidyl-3-(2-pyridyldithio) propionate and reducing the product (usually *in situ*) to the free thiol compound *(8)*, Z = SH [Carlsson *et al, Biochem. J. 173*:723 (1978)].

Thiol derivatives can also be obtained from 3-(halogen-substituted) ethosuximide by replacement of halogen with sulfur nucleophiles [*Preparative Organic Chemistry* 4th ed., Hilgetag and Martini, eds., John Willey and Sons, (New York, 1972), p. 634]. Such halogen derivatives can be prepared as outlined above in sections (a) and (b) by the reaction of the appropriate *bis*-halogen compounds with either *(5)* or *(9)*.

(d) 3-(Maleimido-functionalized) ethosuximides

Ethosuximide derivatives functionalized with maleimido groups can be prepared as outlined in Scheme 4 by reacting the corresponding amino compounds with maleic anhydride to give *(10)* and cyclizing this to form the maleimido derivatives *(11)*. Alternatively, amino derivatives of the drug can be reacted with maleimido-substituted carboxylic acids to give maleimido-substituted ethosuximide *(12)* in which the original linking group R' present in the amines, section (b) above, now has been extended by the linking group R'' and the amide function contributed by the maleimido carboxylic acid. Examples of the latter reagent are known where R'' = phenyl, and linear and branched alkyl [Kitagawa and Aikawa, *J. Biochem. 79:* 233 (1976); Keller and Rudinger, *Helv. Chim. Acta 58*:531 (1975)].

SCHEME 4

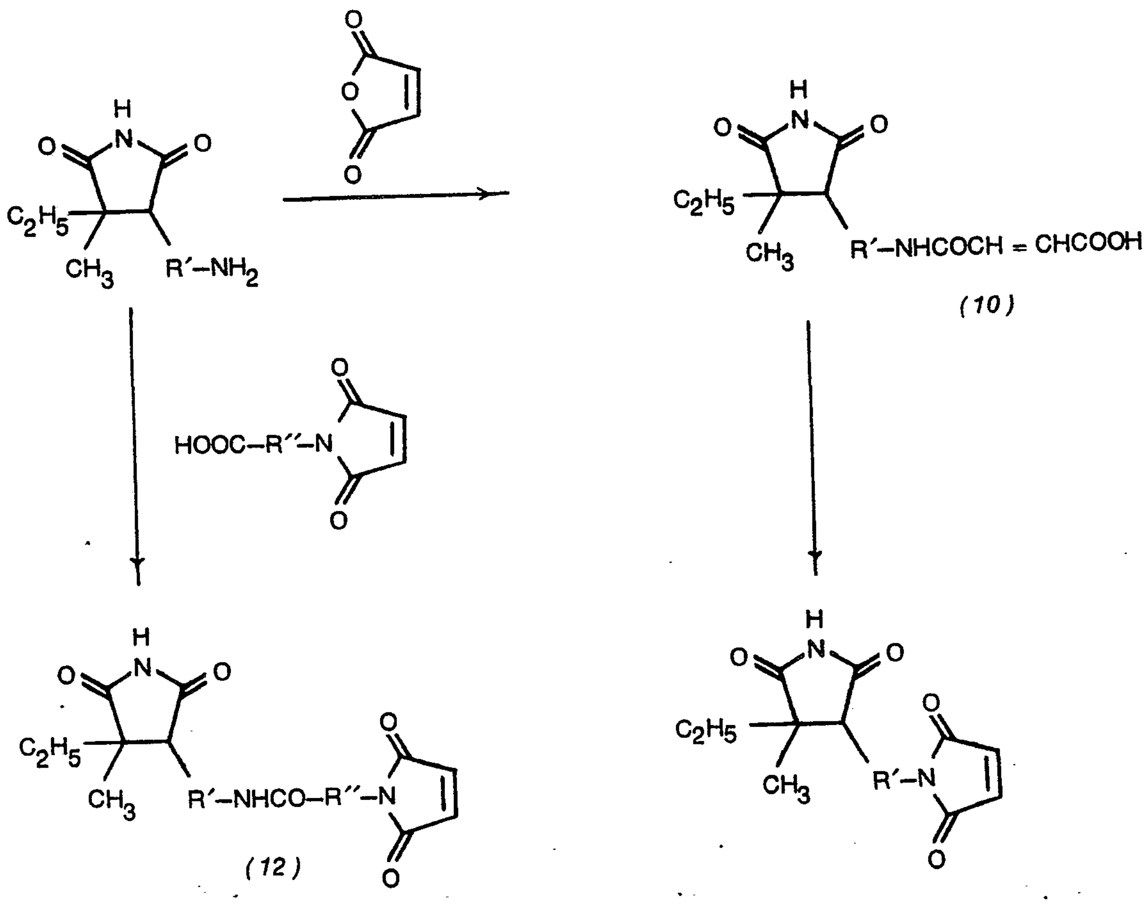

(10)

(12)

(11)

(e) 3-(Hydroxyl-functionalized) ethosuximides

Hydroxy-functionalized ethosuximides can be prepared by reacting intermediates *(5)* and *(9)* with X—R′—Z *(6)*, where X and R′ are as defined above and Z is a hydroxyl group, or more usually, a protected hydroxyl group or a functional group that can be transformed into a hydroxyl group.

For example, *(5)* can be alkylated with X—$(CH_2)_n$—CHO which are available form the class of *omega*-hydroxyaldehydes [Hurd *et al, J. Amer. Chem. Soc. 74*:5324 (1952)], and the products cyclized and reduced to give *(8)* (Z = OH, R′ = $(CH_2)_n$).

From the above-described synthetic routes, it is clear that 3-substituted ethosuximide derivatives of the formula:

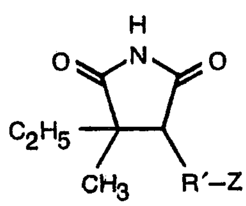

where Z is a reactive group for coupling to the immunogenic carrier material or an appropriate labeling residue (e.g., Z can be amino, carboxyl, hydroxyl, mercapto, or maleimido), can be prepared with a wide latitude in the nature of the linking group R′.

Such derivatives can be coupled to immunogenic carrier materials and labeling substances by available techniques. Carboxyl-containing drug moieties can be attached to the amino groups of carriers and labels by common peptide bond-forming reactions, e.g., those which rely upon activation of the carboxyl group with reagents such as alkyl chloroformates and carbodiimides [*The Peptides* Vol. 1, Gross and Meienhofer, eds., Academic Press (New York, 1979), p. 242, *et seq*], by carbonyl diimidazole (Hilgetag and Martini, *pupra* p.482), or by activator as the acyl azide. Amino-functionalized compounds can be attached to carboxylic acid containing carriers and labels using the amide bond forming reactions cited for

the carboxyl derivatives. In addition, the amino group of the drug moiety can be linked to the amino group of the carrier or label using the following reagents: toluene-2,4-diisocyanate [Hirs and Timasheff, *Methods in Enzymol. 25 (Part B)*:625(1972)]; 4,4′-difluoro-3,3′-dinitrodiphenyl sulfone [Cuatrecasas *et al, J. Biol. Chem. 244*:406 (1969)]; glutaraldehyde (Frohman *et al, Endocrinol. 87*:1055 (1970)]; bi-functional imidoesters [Dutton *et al, Biochem. Biophys. Res. Comm. 23*:730 (1966)] or chlorotriazines [Kay and Crook, *Nature 216*:514 (1967)]. Thiol-containing analogs can be attached to thiol containing carriers or labels by the disulfide exchange procedure [Martin *et al, Biochem. 20*:4229 (1981)]. Alternatively, an amino containing carrier or label can be reacted with a maleimido-substituted carboxylic acid (Keller and Rudinger, *supra*) and this coupled to thiol-functionalized drugs (Kitagawa and Aikawa, *supra*). These compounds can be attached to thiol-containing carriers or labels by the method decribed by Kitagawa and Aikawa, *supra.* Thiol groups can be introduced into amino containing carriers or labels, particularly proteins, by techniques of thiolation described by White, *Methods in Enzymol. 25 (Part B)*:541 (1972).

*Immunoassay Techniques*

The antibodies prepared from the 3-ethosuximide immunogens of the present invention can be used in any immunoassay method, and the corresponding reagent means, for determining ethosuximide, including agglutination techniques, radioimmunoassays, heterogeneous enzyme immunoassays (cf. U.S. Pat. No. 3,654,090), heterogeneous fluorescent immunoassays (cf. U.S. Pats. Nos. 4,201,763; 4,171,311; 4,133,639 and 3,992,631), and homogeneous (separation-free) immunoassays. The latter-most are particularly preferred and include such techniques as fluorescence quenching or enhancement (cf. U.S. Pat. No. 4,160,016), fluorescence polarization (cf. *J. Exp. med. 122*:1029(1965), enzyme substrate-labeled immunoassay (cf. U.S. Pat. No. 4,279,992 and U.K. Pat. Spec. 1,552,607), prosthetic group-labeled immunoassay (cf. U.S. Pat. No. 4,238,565), enzyme modulator-labeled immunoassay, e.g., using inhibitor labels (cf. U.S. Pats. Nos. 4,134,792 and 4,273,866), enzyme-labeled immunoassay (cf. U.S. Pat. No. 3,817,837), energy transfer immunoassay (cf. U.S. Pat. No. 3,996,345), chemically-excited fluorescence immunoassay (cf. U.S. Pat. No. 4,238,195) and double antibody steric hindrance immunoassay (cf. U.S. Pats. Nos. 3,935,074 and 3,998,943).

Moreover, the 3-ethosuximide derivatives of the present invention can be used to prepare the labeled conjugates needed to perform the various immunoassays described above. Appropriate derivatives can be radio-labeled or labeled with fluorescent moieties in accordance with standard methods. Likewise the appropriate labeling moiety for the preferred homogeneous techniques, e.g., an enzyme substrate, a prosthetic group, an enzyme modulator or an enzyme (which is a protein and can be coupled similarly to the immunogenic carrier as described above) can be coupled to the 3-ethosuximide derivatives to yield labeled conjugates.

Particularly preferred labeled conjugates are the ß-galactosyl-umbelliferone-ethosuximide conjugates of the formula:

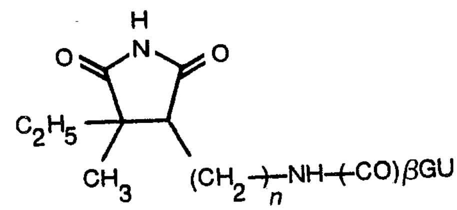

wherein $-(CO)$ßGU is

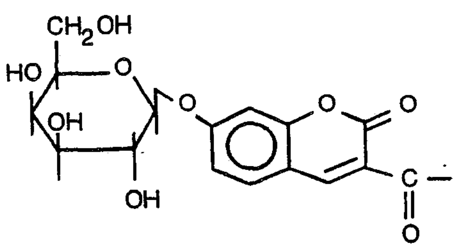

and *n* is an integer from 1 through 10. Such conjugates are prepared by standard peptide condensations of ß-galactosyl-umbelliferone carboxylic acid (U.S. Pat. No. 4,226,978) with the appropriate 3-(aminoalkyl) ethosuximide.

The reagent means of the present invention comprises all of the essential chemical elements required to conduct a desired ethosuximide immunoassay method encompasssed by the present invention. The reagent means is presented in a commercially packaged form, as a composition or admixture where the compatibility of the reagents will allow, in a test device configuration, or as a test kit, i.e., a packaged combination of one or more containers holding the necessary reagents. Included in the reagent means are the reagents appropriate for the binding reaction system desired, e.g., an antibody and labeled conjugate of the present invention. Of course, the reagent means can include other materials as are known in the art

and which may be desirable from a commercial and user standpoint, such as buffers, diluents, standards, and so forth. Particularly preferred is a test kit for the homogeneous competitive binding immunoassay of the present invention comprising (a) an ethosuximide antibody of the present invention and (b) a labeled ethosuximide conjugate which has a detectable property which is altered when bound with the antibody. Also preferred is a test device comprising a reagent composition including an ethosuximide antibody of the present invention and a labeled ethosuximide conjugate which has a detectable property which is altered when bound with the antibody, and a solid carrier member incorporated with the reagent composition. The various forms of such test device are described in EP—A—0051213. The specific label used in the preferred test kit and test device will depend on the technique followed, as described hereinabove.

The present invention will now be illustrated, but is not intended to be limited, by the following examples:

## EXAMPLES

*Reagents*

A. Preparation of ethosuximide derivatives

Ethyl 2-Cyano-3-methyl-2-pentenoate.

A mixture of 50 grams (g) [0.44 mol (Moles)] of ethyl cyanoacetate, 32 g (0.44 mol) of methyl ethyl ketone, 1.3 milliliters (ml) of piperidine, 1.3 ml of glacial acetic acid and 150 ml of toluene was refluxed with a water trap attached for 17 hours. Solvent was removed and the residue distilled under high vacuum to give 60 g of the ester, bp 62—65°C C/0.1 torr.

2-Carbomethoxy-2-(4-N-phthalimidobutyl)-3-ethyl-3-methylsuccinonitrile.

The ethyl ester from above, 20.6 g (0.122 mol), was added to a slurry of 9.2 g (0.122 mol) of potassium cyanide (KCN) in 250 ml of methanol (MeOH) and allowed to stir overnight at room temperature. At this point 34.5 g (0.123 mol) of N-(4-bromobutyl)phthalimide was added and the reaction refluxed under an inert atmosphere for 5 days. It was cooled to room temperature and filtered to remove inorganic material. When cooled overnight the filtrate deposited 17.1 g of the dinitrile as an off-white solid. This substance is the methyl ester and is homogeneous on tlc (9:1 carbon tetrachloride ($CC1_4$): acetone on silica gel plates). An analytical sample was obtained by recrystallization from MeOH to give white crystals, mp 113—115°C.

Analysis: Calculated for $C_{21}H_{23}N_3O_4$: C, 66.13; H, 6.08; N, 11.02.
Found: C, 66.03; H, 6.06; N, 10.70.

2-Ethyl-2-methyl-3-(4-N-phthalimidobutyl) succinimide.

A mixture of 7.7 g (0.02 mol) of the methyl ester dinitrile from above, 2.34 g (0.04 mol) of sodium chloride, 0.54 g (0.03 mol) of water ($H_2O$) and 30 ml dimethyl sulfoxide (DMSO) was heated at exactly 125°C for 165 minutes. The DMSO was then removed over a period of 90 minutes on the rotary evaporator at a bath temperature of 80°C. The residue was dissolved in chloroform ($CHC1_3$) and washed once with $H_2O$. The $H_2O$ wash was back washed with $CHC1_3$. The combined organic extracts were washed with $H_2O$, dried over anhydrous sodium sulfate ($Na_2SO_4$), filtered and evaporated to yield 5.5 g of 2-ethyl-2-methyl-3-(4-phthalimidobutyl)succinonitrile as a light red oil. The $^1H$ NMR spectrum ($CDC1_3$) of this oil possessed the expected peaks: δ 1.5 ($CH_3$ singlet), and δ 1.2 ($CH_3$ triplet). The substance was used without further purification. A solution of 3.6 g of this nitrile in 20 ml of conc. $H_2SO_4$ with 6.6% $H_2O$ was stirred for 2 hours at room temperature, then heated at 100°C for 2.5 hours. It was poured over 100 g of ice, and when the ice had melted, the mixture was extracted twice with $CHC1_3$. The combined organic etracts were washed with $H_2O$, dried and evaporated. The residue was chromatographed on 100 g of silica gel eluting with 2.5% acetone in $CHC1_3$ (39:1 v:v); 17 ml fractions were collected. Fractions 75—100 were combined and evaporated to give 950 milligrams (mg) of the *bis*-imide as a thick oil.

Analsysis: Mass Spectrum (70 e.V.) *m/e* 342 [$M^+$];313 [$M^+$ minus $C_2H_5$]
$^1H$ NMR Spectrum ($CDC1_3$);
δ 7.8 (aromatic multiplet),
δ 3.8 ($CH_2$ broad triplet),
δ 2.6 (CH, broad multiplet),
δ 1.3 1.2 ($CH_3$, doublet),
δ 0.9 ($CH_3$, triplet).

2-(4-Aminobutyl)-3-ethyl-3-methylsuccinimide.

A solution of 3.29 g (0.01 mol) of the *bis*-imide from above and 370 mg of 85% hydrazine in 50 ml of absolute ethanol was heated to reflux for 45 minutes. An additional 50 mg (0.05 ml) of hydrazine was added and heating continued for 30 minutes. When cool the solvent was evaporated and the residue chromatographed on 250 g of silica gel eluting with 60:10:1 (v/v/v) $CHC1_3$:MeOH:conc. ammonium hydroxide ($HN_4OH$). Fourteen ml fractions were collected. Fractions 83—140 were pooled and evaporated

to give 1.5 g of the 3-amino ethosuximide derivative as a clear oil. The $^1H$ NMR spectrum of this oil showed no aromatic protons and the expeced multiplets for the aliphatic CH groups. A small amount was converted to a powdery HC1 salt, mp 140—148°C from ethanol.

Analysis: Calculated for $C_{11}H_{20}N_2O_2 \cdot HC1$: C, 53.11; H, 8.51; N, 11.26.
Found: C, 52.96; H, 8.51; N, 11.28.

B. Preparation of labeled ethosuximide conjugate

2-[4-(7-ß-Galactosylcoumarin-3-carboxyamido)butyl]-3-ethyl-3-methyl-succinimide.

A mixture of 7-ß-galactosylcoumarin-3-carboxylic acid (400 mg, 1 mmol) [U.S. Pat. No. 4,226,978], 120 mg (1 mmol.) of N-hydroxysuccinimide, and 200 mg (1 mmol) of dicyclohexylcarbodiimide in 5 ml of dry DMF was stirred at 0°C for 15 minutes, then allowed to warm to room temperature. After 4 hours it was filtered and the filtrate added dropwise (over 5 minutes at −5°C) to a second solution made by dissolving 195 mg (1 mmol) of the HC1 salt of the 3-amino ethosuximide derivative from Part A above and 170 mg (2 mmol) of sodium bicarbonate ($NaHCO_2$) in a 4 ml of $H_2O$. the combined solution was stirred for 1 hr in the ice bath, then allowed to stand overnight in the refrigerator. Ten grams of silica gel was added and the solvent removed under high vacuum. The impregnated adsorbent was placed atop at column of 100g of silica gel made up in ethyl acetate. The column was eluted with 1 L of ethyl acetate (EtOAc), then with a linear gradient of 2 L EtOAc to 2 L of ethanol (EtOH). The appropriate fractions were pooled and evaporated to give 120 mg of the labeled conjugate as a white solid, mp 157—161°C.

Analysis: Calculated for $C_{27}H_{34}N_2O_{11} \cdot H_2O$: C, 55.87; H, 6.25; N, 4.82.
Found: C, 55.52; H, 6.10; N, 4.71.

C. Preparation of ethosuximide immunogen and antibodies

Ten (10) ml of distilled water (5°C) was placed in a test tube with a small magnetic stir bar. Using gentle stirring with a magnetic stirrer, 96.1 mg (0.39 mmol) of 2-(4-aminobutyl)-3-ethyl-3-methyl succinimide, *supra,* was dissolved in water. The pH of the solution (~2.5) was adjusted to 4.5 by dropwise addition of 1 M sodium hydroxide (NaOH). The solution was then cooled to 5°C in an ice water bath. With gentle stirring 100 mg (1.52 μmole) of bovine serum albumin (BSA) was slowly added in small batches to the cold ethosuximide derivative solution, making sure that each batch was completely dissolved before more BSA was added. After all of the BSA was dissolved, the pH of the solution was checked and readjusted to pH 4.5 if necessary. Again with gentle stirring 270 mg (1.4 necessary. Again with gentle stirring 270 mg (1.4 mmole) of 1-ethyl-3-(3-dimethylaminopropyl)carbodiimide hydrochloride (EDC) was slowly added (all of the EDC was added within one minute to the cold solution mixture. This solution was gently stirred at 4°C overnight in the cold room. The next morning the reaction mixture was warmed to room temperature prior to application to a Sephadex® G-25 column.

The reaction mixture was applied to a column (3.0 × 55 cm) of Sephadex® G-25 (fine) (Pharmacia, Piscataway, New Jersey USA) which had previously been equilibrated with 5 mM sodium phosphate, pH 7.0. Fractions of 4.3 ml were collected and the absorbance at 280 nm was monitored. The ethosuximide-BSA conjugate eluted in the void volume of the column and these column fractions (25—30) were pooled. The protein concentration of the immunogen pool was then determined using an appropriate method [e.g., Lowry *et al, J. Biol. Chem. 193*:265(1957)] and if the protein concentration was greater than 1 mg/ml the immunogen was diluted to 1 mg/ml with saline (0.85% NaC1).

Six milliliters of immunogen (1 mg/ml) was combined with 12 ml of Freunds complete adjuvant and 6 ml of saline. Rabbits were immunized subcutaneously each with 2 ml of this mixture. Three weeks later they were reimmunized with the same mixture prepared with incomplete Freunds adjuvant. The booster immunizations were repeated every 4 weeks. Test bleedings were taken one week after the boosters. Antiserum with suitable titers were obtained by 6 months after the initial immunization.

Immunoassay

A homogeneous substrate-labeled fluorescent immunoassay (SLFIA — U.S. Patent No. 4,279,992) for ethosuximide was established as follows:

A. Reagents

1. Antibody/Enzyme Reagent — 50 mM Bicine buffer [N,N-*bis*-(2-hydroxyethyl)glycine, Calbiochem-Behring Corp., LaJolla, California USA], pH 8.5, containing 1.5 units/ml ß-galactosidase, sufficient antiserum raised against the ethosuximide immunogen (*Reagents,* Part C above) to decrease fluorescence to 5% of that in the absence of antiserum, and 15.4 mM sodium azide.

2. Conjugate Reagent — 30 mM formate buffer, pH 3.5, containing 0.001% (v/v) Tween® 20, and 0.19 μM (micromolar) ß-GU-ethosuximide (*Reagents,* Part B above) and 15.4 mM sodium azide.

3. Ethosuximide Standards — USP reference standard ethosuximide added to normal human serum; diluted 51-fold with 50 mM Bicine buffer, containing 15.4 mM sodium azide.

B. Assay Method

To 3 ml volumes of the Antibody/Enzyme Reagent in cuvettes were added 100 μl (microliters) of the diluted Ethosuximide Standards. Then to begin the reaction, 100 μl of the Conjugate Reagent was added to each cuvette with mixing. After 20 minutes the fluorescence intensity was measured in each cuvette (excitation 400 nm, emission 450 nm).

C. Results

Performance of the assay yielded the following results:

| Ethosuximide (mg/ml) | Normalized Fluorescence Units |
|---|---|
| 0 | 39.0 |
| 20 | 50.5 |
| 50 | 64.0 |
| 100 | 80.0 |
| 150 | 90.0 |

The immunoassay could be used to determine ethosuximide concentrations in serum samples.

**Claims**

1. An ethosuximide immunogen comprising ethosuximide covalently linked at its 3-position to an immunogenic carrier material, preferably a protein or polypeptide.

2. An ethosuximide immunogen according to claim 1 of the formula:

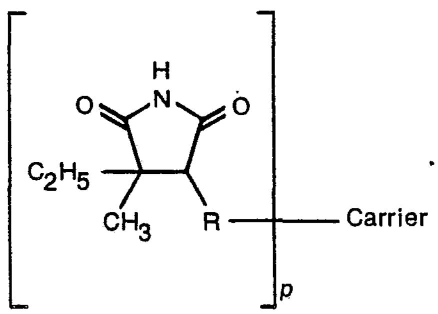

wherein R is a linking group, Carrier is the immunogenic carrier material, and p is on the average from 1 to 50, preferably from 1 to 25.

3. An ethosuximide immunogen according to claim 2 of the formula:

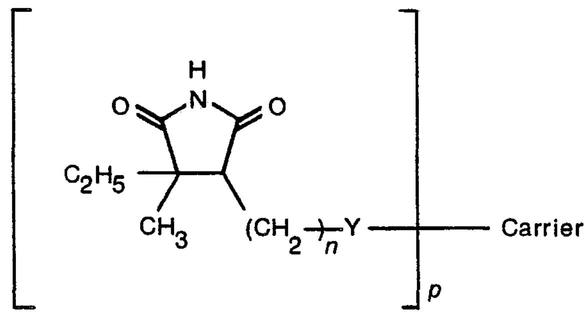

wherein Y is an amide group, n is an integer from 1 to 10, preferably 4, and p is on the average from 1 to the number of available amide coupling sites on said carrier material.

4. An antibody prepared against an immunogen according to any of Claims 1 to 3.

5. An ethosuximide derivative of the formula:

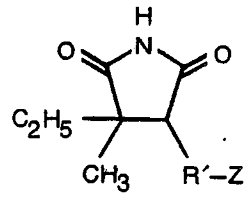

wherein R′ is a linking group, preferably a group of the formula $-(CH_2)_n-$, wherein n is an integer from 1 to 10, and Z is amino, carboxyl, thiol, maleimido, or hydroxyl, preferably carboxyl or amino.

6. An immunoassay method for determining ethosuximide, characterized in that the antibody of Claim 4 is employed as the antibody to ethosuximide.

7. Reagent means for determining ethosuximide by immunoassay, characterized in that the antibody of Claim 4 is employed as the antibody to ethosuximide.

8. A test kit for determining ethosuximide by homogeneous immunoassay, comprising
(a) the antibody of Claim 4 and
(b) a labeled ethosuximide conjugate which has detectable property which is altered when bound with said antibody.

9. A test device for determining ethosuximide by homogeneous immunoassay, comprising
(a) a reagent composition including the antibody of Claim 4 and a labeled ethosuximide conjugate which has a detectable property which is altered when bound with said antibody, and
(b) a solid carrier member incoporated with said reagent composition.

10. A ß-galactosyl-umbelliferone-ethosuximide conjugate of the formula

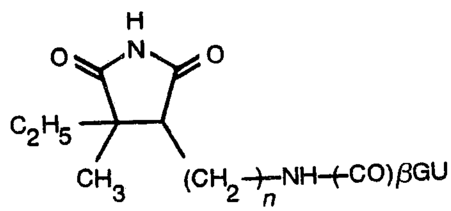

wherein (–CO)ßGU is

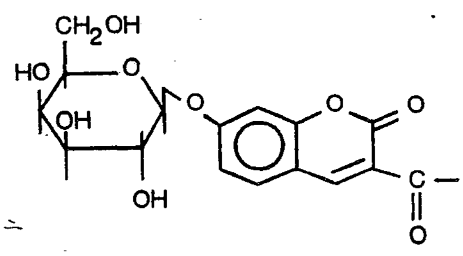

and n is an integer from 1 to 10, preferably 4.

**Patentansprüche**

1. Ethosuximid-Immunogen, umfassend Ethosuximid, kovalent gebunden in seiner 3-Stellung an ein immunisierendes Trägermaterial, vorzugsweise ein Protein oder Polypeptid.

2. Ethosuximid-Immunogen gemäss Anspruch 1 der Formel

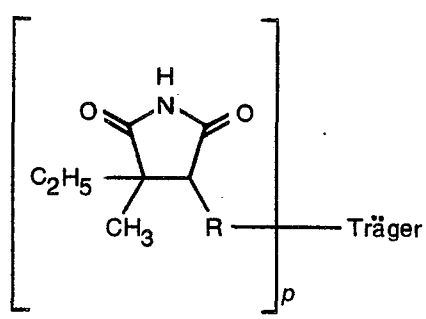

worin R eine verbindende Gruppe ist, "Träger" ein immunisierendes Trägematerial bedeutet und p im Durchscnitt 1 bis 50, vorzugsweise 1 bis 25 ist.

3. Ethosuximid-Immunogen gemäss Anspruch 1 der Formel

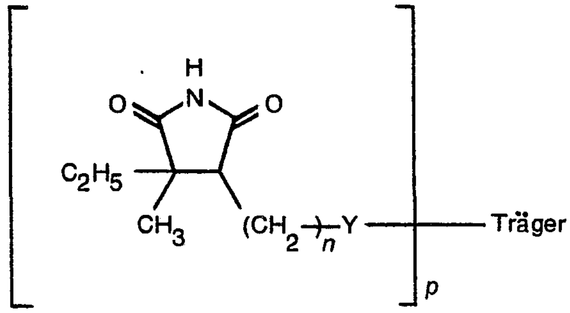

worin
Y eine Amidgruppe,
n eine ganze Zahl von 1 bis 10, vorzugsweise 4, und
p im Durchschnitt 1 bis zu der Zahl der verfügbaren Amidkupplungsstellen an dem Trägermaterial bedeuten.

4. Antikörper, hergestellt gegen ein Immunogen gemäss einem der Ansprüche 1 bis 3.

5. Ethosuximidderivat der Formel

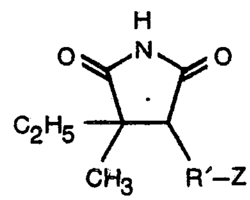

worin
R' eine verbindende Gruppe, vorzugsweise eine Gruppe der Formel $-(CH)-_n$, worin n eine ganze Zahl von 1 bis 10 ist, und
Z Amino, Carboxyl, Thiol, Maleimido oder Hydroxyl, vorzugsweise Carboxyl oder Amino, bedeuten.

6. Immunoassay-Methode für die Bestimmung von Ethosuximid, dadurch gekennzeichnet, dass man den Antikörper gemäss Anspruch 4 als Antikörper Für Ethosuximid verwendet.

7. Reagens für die Bestimmung von Ethosuximid durch Immunoassay, dadurch gekennzeichnet, dass der Antikörper von Anspruch 4 als Antikörper für Ethosuximid verwendet wird.

8. Testeinrichtung für die Bestimmung von Ethosuximid durch homogene Immunoassay, umfassend
(a) den Antikörper von Anspruch 4 und
(b) ein markiertes Ethosuximid-Konjugat, welches eine nachweisbare Eigenschaft, die sich beim Binden mit dem Antikörper ändert, hat.

9. Testeinrichtung für die Bestimmung von Ethosuximid durch homogene Immunoassay, umfassend
(a) eine Reagenszusammensetzung, die den Antikörper von Anspruch 4 und ein markiertes Ethosuximid-Konjugat, welches eine nachweisbare Eigenschaft, aufweist, die sich beim Binden mit dem Antikörper verändert, umfasst, und
(b) ein festes Trägerteil, welches mit der Reagenszusammensetzung inkorporiert ist.

10. ß-Galactosyl-umbelliferon-ethosuximid-Konjugat der Formel

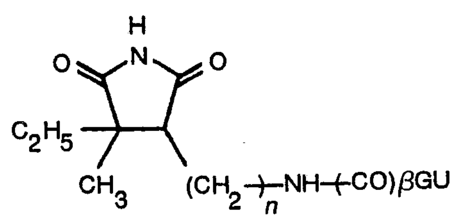

worin —(CO)ßGU

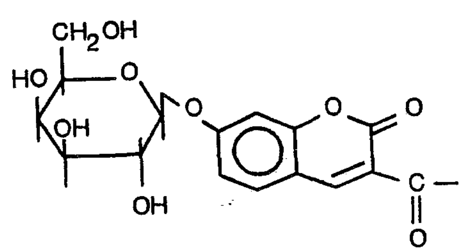

bedeutet und n eine ganze Zahl von 1 bis 10, vorzugsweise 4, bedeutet.

**Revendications**

1. Immunogène d'éthosuximide, comprenant de l'éthosuximide lié pa covalence, à sa position 3, à une matière immunogène de support, di préférence une protéine ou un polypeptide.

2. Immunogène d'éthosuximide selon la revendication 1, de formule

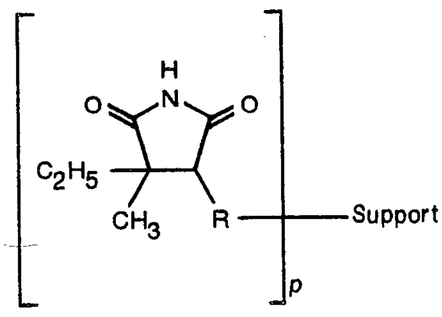

dans laquelle
R est un groupe de liaison,
"Support" est la matière immunogène de support, et
p vaut en moyenne 1 à 50, de préférence 1 à 25.

3. Immunogène d'éthosuximide selon la revendication 2, de formule

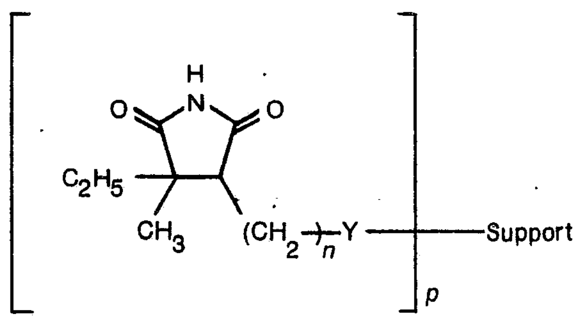

dans laquelle
Y représente un groupe amide,
n est un nombre valant 1 à 10, et valant de préférence 4, et
p vaut en moyenne 1 jusqu'au nombre de sites, disponibles sur ladite matière de support, pour le couplage par fonction amide.

4. Anticorps préparé contre un immunogène selon l'une l'une quelconque des revendications 1 à 3.

5. Dérivé d'éthosuximide de formule:

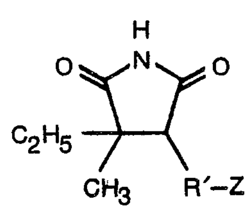

dans laquelle
R' est un groupe de liaison, de préférence un groupe de formule $(CH_2)_n$, dans laquelle n est un nombre entier valant 1 à 10, et
Z représente un groupe amino, carboxyle, thiol, maléimido ou hydroxyle, de préférence carboxyl ou amino.

6. Procédé de dosage immunologique pour déterminer l'éthosuximide, caractérisé en ce qu'on utilise l'anticorps de la revendication 4 comme anticorps de l'éthosuximide.

7. Réactif global pour déterminer l'éthosuximide par dosage immunologique, caractérisé en ce qu'on utilise l'anticorps de la revendication 4 comme anticorps de l'éthosuximide.

8. Necessaire d'essai pour déterminer l'éthosuximide par dosage immunologique homogène, comprenant:

(a) l'anticorps de la revendication 4, et
(b) un conjugué d'éthosuximide marque, qui possède une propriété décelable et qui est modifiée quand le conjugué est lié audit anticorps.

9. Dispositif d'essai pour déterminer l'éthosuximide par dosage immunologique homogène, comprenant:
(a) une composition de réactifs comprenant l'anticorps de la revendication 4 et un conjugué d'éthosuximide marqué qui possède une propriété décelable modifiée en cas de liaison avec ledit anticorps, et
(b) un support solide incorporé à ladite composition de réactif.

10. Conjugué de ß-galactosyl-ombelliférone/éthosuximide, de formule

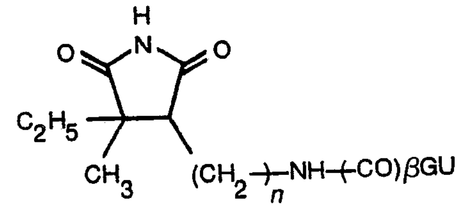

dans laquelle —(CO)ßGU représente

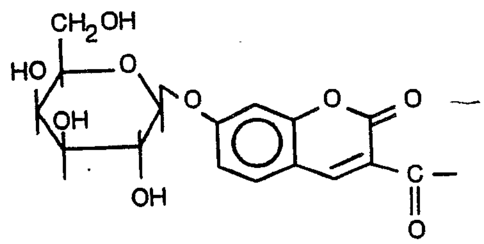

et n est un nombre entier valant 1 à 10, et valant de préférence 4.